# EUROPEAN PATENT SPECIFICATION

(11) **EP 2 497 430 B1**
(45) Date of publication and mention of the grant of the patent: **09.05.2018**
(21) Application number: 10827787.2
(22) Date of filing: 25.10.2010
(51) Int. Cl.: A61B 17/08, A61B 17/326, A61B 17/00, A61F 13/14

(54) **FORESKIN-PLASTER AND FIXATOR FOR CIRCUMCISION SURGERY**
VORHAUTPFLASTER UND FIXIERER FÜR ZIRKUMZISIONSCHIRURGIE
PANSEMENT POUR PRÉPUCE ET FIXATEUR UTILISABLES EN CHIRURGIE DE LA CIRCONCISION

(30) Priority: 03.11.2009 CN 200920266030 U
(43) Date of publication of application: 12.09.2012
(73) Proprietor: Shang, Jianzhong, Anhui 241001 (CN)
(72) Inventor: Shang, Jianzhong, Anhui 241001 (CN)
(74) Representative: Harris, Oliver John Richard
(86) International application number: PCT/CN2010/001675
(87) International publication number: WO 2011/054173

(56) References cited:
- WO-A1-2007/133210
- WO-A2-03/020106
- CN-A- 101 889 923
- CN-U- 201 572 128
- CN-Y- 2 155 837
- CN-Y- 2 240 346
- CN-Y- 2 494 183
- CN-Y- 2 817 743
- CN-Y- 200 960 224
- US-A- 1 765 319
- US-A- 5 163 943
- US-A- 5 269 788
- US-A1- 2004 054 405
- US-B2- 6 660 012

## Description

### Field of the Invention

The invention relates to a type of medical apparatus, especially the provision of a type of foreskin plaster patch and a fixator for use in circumcision surgery.

### Background and Prior Art

Redundant prepuce or phimosis is one of the origins of male urinary system infection and sexually transmitted diseases. Redundant prepuce or phimosis can cause urinary tract infection that will lead to chronic prostatitis, which will incur a series of symptoms like back pain, impotence, prospermia, etc. Therefore, removing redundant foreskin or phimosis is known as a good measure to prevent these sort of diseases.

Regarding the removal of redundant foreskin or phimosis, otherwise known as circumcision, there have been numerous medical advances over the years , but a circumcision operation will inevitably lead to bleeding, pain and hospitalization. Circumcision is one of the traditional measures to treat phimosis and redundant prepuce, performed by medical specialists such as urologists. Typically, circumcision involves three surgical steps comprising redundant foreskin removal, hemostasia, and applying an apposition suture at the incisal edge. After the operation, the patient generally cannot walk, and he will typically feel intolerable pain at each dressing change and further may suffer great pain when stitches are removed. Furthermore, if hemostasis by ligation is done by halves, it may cause preputial hematoma, requiring perhaps another surgical treatment. Although laser and high-frequency electric-knife technology has been used in foreskin circumcisions, their main advantage is only that they eliminate the use of scissors to make incisions and cauterize the point of bleeding. Such a therapeutic method is likely to subject the patient to tissular burn and infection, while the cost for infection prevention is very high. Judging from comparisons among various provinces and cities, whenever laser circumcision is performed, antibiotic treatment after the operation is common for 5-7 days, plus additional antibiotics are usually taken orally for an addition 5-7 days, while the cost generally ranges from 150 to 360 yuan, with the total cost of treatment is at least 1000-1500 yuan.

In addition to the above-mentioned method of laser circumcision, using an electric-knife for circumcising a penis is very common now. Similar to the laser circumcision, it can remove redundant foreskin from the glans to achieve an appropriate length of foreskin.

However, the capillary vessel at the wound site typically heals slowly, causing discomfort at the site of the foreskin cut. The risk of infection in such a circumstance is high, with an increased likelihood of pain for the patient.

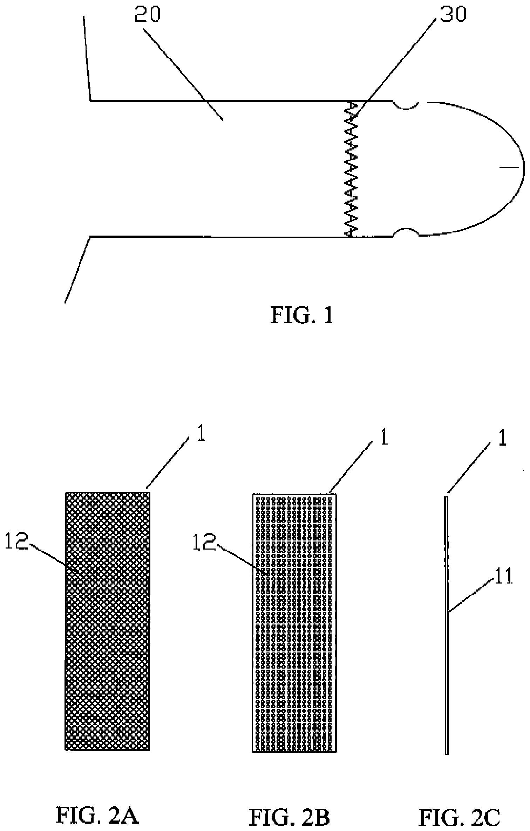

Fig 1 illustrates one, prior-art method of performing circumcision surgery. The steps involved in this kind of surgery comprise: removing a middle section of redundant foreskin, bringing the two foreskin edges together, and then suturing them together to achieve a desired foreskin length. In this way, both sides of the capillary vessel at the wound are in contact and, thus, heal quickly.

However, this form of surgery requires a doctor to stitch the joint 30 of the two sides of the foreskin. The surgery is very difficult and it is hard to achieve a good result with respect to healing of the foreskin.
WO2007/133210 discloses a dressing for use inter alia in bandaging a penis, comprising a sleeve with a lining which is substantially non-adherent to the part being bandaged and peripheral adhesive portions for securing the dressing in use. The skin contacting surface may comprise a breathable transdermal device.

### Summary of the Invention

The main purpose of this invention lies in overcoming the deficiencies of the existing technology and providing a foreskin plaster patch and a fixator for circumcision surgery.

In order to realize the abovementioned purpose, this invention adopts a technical solution in the form of an apparatus comprising a foreskin plaster patch and a fixator, wherein the foreskin plaster patch is in the form of an elongated strip, having an adhesive surface on one side, and has a ventilation structure. Preferably, the ventilation structure comprises a plurality of breather holes or breathable meshes. The fixator comprises an annular sleeve, which has an accommodation space to accommodate penis. A plurality of fixing pins are provided on the outer surface of the fixator. Preferably, the fixing pins are needle-like structures vertically located on the outer surface of the fixator. Preferably, the fixator is integrally formed from materials absorbable by human body.

The advantageous effects of the invention are as follows:
1. The foreskin plaster patch is used in conjunction with the fixator. As a result, there is no need to use stitches during the circumcision surgery. Therefore, the surgical process is simplified, and the incision will heal to have the best visual effects.
2. The ventilation structure on the foreskin plaster patch, e.g., the breather holes or breathable meshes, can reduce the high risk of infection.
3. The fixing pins on the outer surface of the fixator can secure the foreskin of both sides of the incision to bring them close together with each other and the plaster patch can easily be placed on the foreskin. The structure of the fixator is simple and reliable.
4. The fixator is integrally formed from materials absorbable by human body. There is no need to remove the fixator after surgery.

Further novel features and other objects of the present invention will become apparent from the following detailed description, discussion and claims, taken in conjunction with the drawings.

### Brief Description of the Drawings

Fig 1 illustrates a penis circumcised using conventional circumcision method;
Fig 2A illustrates one embodiment of a foreskin plaster patch of the present invention;
Fig 2B illustrates another embodiment of the foreskin plaster patch of Fig 1;
Fig 2C illustrates a side view of the foreskin plaster patch of the present invention;
Fig 3A illustrates a fixator used in conjunction with the foreskin plaster patch shown in Figs 2A - 2C;
Fig 3B illustrates the side view of the fixator shown in Fig 3A;
Fig 3C illustrates an enlarged view of the surface of the fixator shown in Fig 3A;
Fig 4 illustrates a perspective view of the fixator used to secure two circumferential foreskin edges together after removal of a middle section of foreskin; and
Fig 5 illustrates a perspective view of the foreskin plaster patch placed over the fixator shown in Fig 4 after removal of the middle section of foreskin.

### Detailed Description of the Preferred Embodiments of the Invention

The foreskin plaster patch and the fixator mentioned in this invention are used in conjunction with each other. In the surgical field of circumcision, there is no prior art that teaches or suggests the use of these two devices together to heal the foreskin that has been cut during a circumcision.

As illustrated in Figs. 2A to 2C, the present invention is related to a foreskin plaster patch used in foreskin circumcision surgery. The foreskin plaster patch 1 is in the form of an elongate strip, having an adhesive surface 11 on one side, further comprising a ventilation structure 12. The ventilation structure may comprise breathable meshes in one embodiment, as illustrated in Fig. 2A, and may alternatively comprise breather holes in a second embodiment, as illustrated in Fig. 2B.

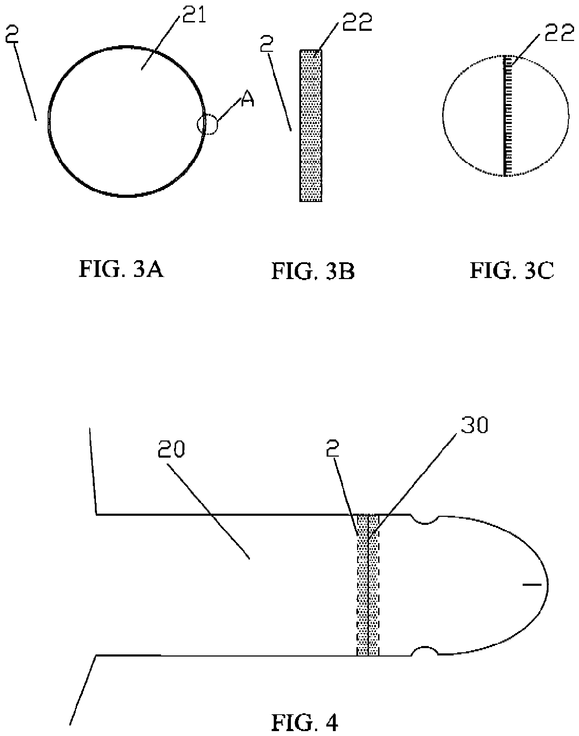

Next, referring to Fig. 3A and 3B, a fixator 2 is shown, used in conjunction with the foreskin plaster patch 1 for circumcision surgery. The fixator 2 is an annular sleeve, which has an accommodation space 21 to accommodate penis. A plurality of fixing pins 22 are provided on the outer surface of the fixator 2. In the embodiments illustrated in Fig. 3B and 3C, the fixing pins are needle-like structures vertically located on the outer surface of the fixator 2, and the circumferential foreskin edges resulting from removal of a section of foreskin can be fixed in close relation to each other by means of the small pins.

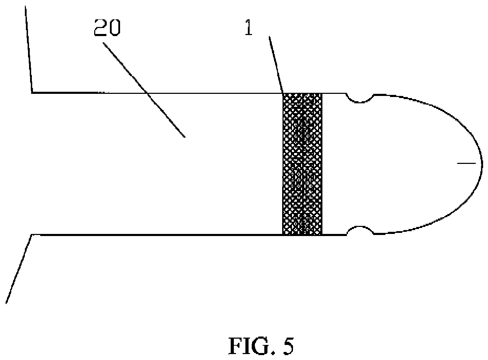

Fig. 4 illustrates a perspective view of fixator 2 in place, used to secure two circumferential foreskin edges together after removal of a middle section of foreskin. During the surgery, a strip of foreskin in the middle is cut by using one of several standard surgical techniques. The fixator 2 is sleeved on the penis 20, so that the penis is contained within the containing space 21 and the edges of the foreskin at each end is fixed by the fixing pins 22 provided on the outer surface of the fixator 2. The small pins acts to pierce the foreskin such that the two edges of each piece of foreskin are fixed with respect to each other. After the foreskin is fixed by fixator 2, the connection portions 30 of two segments of the cut foreskin are protected by using the foreskin plaster patch 1, and the goal of enhanced fixation is achieved, as shown in Fig 5.

Using the above-mentioned foreskin plaster patch 1 and fixator 2, circumcision surgery is made more convenient and efficient, with faster healing times, and no stitches to remove. Additionally, surgical process is simplified. After surgery, the foreskin plaster patch 1 can be simply torn off. Comparatively, the fixator 2 is not easily torn off, however it is made of a biological material that can be absorbed by the body. Therefore, there is no need to remove the fixator 2 after surgery, as the biological material is absorbed during the healing process. Such medical absorbability materials have been widely used.

To sum up, the present invention is expounded from the aspects of its application target, effectivity, evolutionarity and novelty, etc. The above-mentioned implementation, explained with respect to the figures, is considered to be one preferred implementation example of the invention, but should not be considered limiting.

## Claims

1. An apparatus for use in circumcision surgery, comprising:
a foreskin plaster patch (1) in the form of an elongated strip, having an adhesive surface (11) on one side and further comprising a ventilation structure (12);
and **characterised in that** the apparatus further comprises:
a fixator (2) comprising an annular sleeve comprising an accommodation space (21) to accommodate a penis and a plurality of fixing pins (22) located on an outer surface of the fixator.

2. The apparatus of claim 1, wherein the ventilation structure (12) comprises a plurality of breather holes.

3. The apparatus of claim 1, wherein the ventilation structure (12) comprises a breathable mesh.

4. The apparatus of claim 1, wherein the fixing pins (22) are needle-like structures extending outwardly from the outer surface of the fixator.

5. The apparatus of claim 1, wherin the fixator is integrally formed from a material or materials absorbable by a human body.

## Patentansprüche

1. Vorrichtung zur Verwendung in einem chirurgischen Beschneidungseingriff, die Folgendes umfasst:
ein Vorhautpflasterstück (1) in der Form eines länglichen Streifens, das eine Klebefläche (11) auf einer Seite aufweist und weiterhin eine Ventilationsstruktur (12) umfasst;
und **dadurch gekennzeichnet, dass** die Vorrichtung weiterhin Folgendes umfasst:
einen Fixator (2), der eine ringförmige Hülse umfasst, die einen Aufnahmeraum (21), um einen Penis aufzunehmen, und mehrere Fixierstifte (22) umfasst, die sich auf einer Außenfläche des Fixators befinden.

2. Vorrichtung nach Anspruch 1, wobei die Ventilationsstruktur (12) mehrere Atmungslöcher umfasst.

3. Vorrichtung nach Anspruch 1, wobei die Ventilationsstruktur (12) ein atmungsaktives Geflecht umfasst.

4. Vorrichtung nach Anspruch 1, wobei die Fixierstifte (22) nadelartige Strukturen sind, die sich von der Außenfläche des Fixators nach außen erstrecken.

5. Vorrichtung nach Anspruch 1, wobei der Fixator integral aus einem Material oder Materialien, die durch einen menschlichen Körper absorbierbar sind, hergestellt ist.

## Revendications

1. Appareil destiné à être utilisé lors d'interventions chirurgicales de circoncision, comportant :
un pansement pour prépuce (1) sous la forme d'une bande allongée, comprenant une surface adhésive (11) d'un côté et comprenant en outre une structure aérée (12) ;
et **caractérisé en ce que** l'appareil comporte en outre :
un dispositif de fixation (2) comprenant un manchon annulaire doté d'un espace de mise en place (21) pour loger un pénis et d'une pluralité de tiges de fixation (22) situées sur une surface extérieure du dispositif de fixation.

2. Appareil selon la revendication 1, dans lequel :
la structure aérée (12) comporte une pluralité d'orifices de respiration.

3. Appareil selon la revendication 1, dans lequel :
la structure aérée (12) comporte une maille aérée.

4. Appareil selon la revendication 1, dans lequel :
les tiges de fixation (22) sont des structures semblables à des aiguilles s'étendant vers l'extérieur à partir de la surface extérieure du dispositif de fixation.

5. Appareil selon la revendication 1, dans lequel :
le dispositif de fixation est intégralement constitué d'une matière ou de matières absorbable(s) par le corps humain.
